# EUROPEAN PATENT APPLICATION

(11) **EP 2 058 304 A1**
(43) Date of publication of application: **13.05.2009**
(21) Application number: 07806431.8
(22) Date of filing: 24.08.2007
(51) Int. Cl.: C07D 243/24, A61K 31/5513, A61P 25/04, A61P 43/00

(54) **P2X4 RECEPTOR ANTAGONIST**

(30) Priority: 25.08.2006 JP 2006228747
(71) Applicant: Nippon Chemiphar Co., Ltd., Chiyoda-ku Tokyo 1010032 (JP)
(72) Inventor: SAKUMA, Shogo, Yoshikawa-shi, Saitama 3420041 (JP); ENDO, Tsuyoshi, Tokyo 1570077 (JP); IMAI, Toshiyasu, Tokyo 1200031 (JP); KANAKUBO, Noriko, Koga-shi, Ibaraki 3060025 (JP); ARAI, Masahiko, Koshigaya-shi, Saitama 3430041 (JP); TAKAHASHI, Toshihiro, Misato-shi, Saitama 3410003 (JP); YAMAKAWA, Tomio, Kashiwa-shi, Chiba 2770884 (JP); TSUDA, Makoto, Kyushu University, Fukuoka-shi, Fukuoka 8128581 (JP); INOUE, Kazuhide, Kyushu University, Fukuoka-shi, Fukuoka 8128581 (JP)
(74) Representative: Albrecht, Thomas
(86) International application number: PCT/JP2007/066954
(87) International publication number: WO 2008/023847

(57) **Abstract**

A compound having the following formula (II) or its pharmacologically acceptable salt is used as a P2X₄ receptor antagonist: 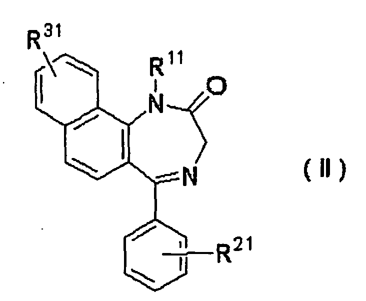 in which
R¹¹ represents hydrogen or an alkyl group having 1-8 carbon atoms;
R²¹ represents an alkyl group having 1-8 carbon atoms, an alkoxy group having 1-8 carbon atoms, an alkyl group having 1-8 carbon atoms and having 1-3 halogen substituents, or hydroxyl; and
R³¹ is hydrogen or a halogen atom.

## Description

### [FIELD OF THE INVENTION]

The present invention relates to 1,4-diazepin-2-one derivatives showing P2X₄ receptor antagonism.

### [BACKGROUND OF THE INVENTION]

ATP receptors are basically classified into P2X family of the ion-channel type receptor and P2Y family of the protein-coupled receptor. Until now, there are reported, respectively, seven sub-types (P2X₁₋₇) and eight sub-types (P2Y_{1, 2, 4, 6, 11-14}).

It has been reported that P2X₄ receptor (Genebank No. X87763) which is a sub-type of P2X family is present widely in the central nervous systems:
Non-patent publication 1: Buell, et al. (1996) EMBO J. 15:55-62;
Non-patent publication 2: Seguela et al. (1996) J. Neurosci. 16:448-455;
Non-patent publication 3: Bo, et al. (1995) FEBS Lett. 375:129-133;
Non-patent publication 4: Soto, et al. (1996) Proc. Natl. Acad. Sci. USA 93:3684-3788;
Non-patent publication 5: Wang, et al. (1996) Biochem. Res. Commun. 220:196-202.

The mechanism of pathogenesis of intractable pains such as neuropathic pain is not unclear. Thus, if non-steroidal anti-inflammatory drugs (NSAIDs) or morphine are less effective in patients, there are no other way of pharmacotherapy.

The neuropathic pain is caused by injury of peripheral or central nervous systems, for instance, post-surgery pain, spinal cord injury, herpes zoster or trigeminal neuralgia.

Recently, Inoue, et al. examined the involvement of P2X receptor in neuropathic pain using dorsal root ganglion neuron-injured animal models which induce allodynia, and suggested that the nerve-injured pain (particularly, allodynia) is caused via P2X₄ receptors on spinal microglia:
Non-patent publication 6: M. Tsuda, et al. (2003) Nature, 424,778-783;
Non-patent publication 7: Jeffrey A.M. Coull, et al. (2005) Nature, 438, 1017-1021; and
Patent publication 1: United States patent publication 20050074819.

Accordingly, compounds enabling to inhibit the action of P2X₄ receptor can be expected to be employed for preventing or treating pains such as nociceptive pains, inflammatory pains and neuropathic pains.

Patent publication 2 (WO 2004/085440) discloses that benzofuro-1,4-diazepin-2-one derivatives having the below-illustrated formula (A) show P2X₄ receptor antagonism: 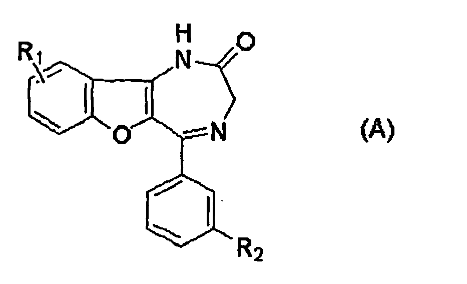 in which R₁ is halogen and R₂ is hydrogen, halogen, nitro, cyano, C(O)-OR₃, C(O)-NR₄R₅, SO₂-OR₃ or SO₂-NR₄R₆, or R₁ is hydrogen and R₂ is halogen, nitro, cyano, C(O)-OR₃, C(O)-NR₄R₅, SO₂-OR₃ or SO₂-NR₄R₆.

Non-patent publication 8 (Journal of Medicinal Chemistry (1994), 37(6), 745-57) and Non-patent publication 9 (Medicinal Chemistry Research (1996), 6(6), 384-391) disclose a compound of the following formula (B), which appears to be analogous to the below-mentioned compound of the present invention: 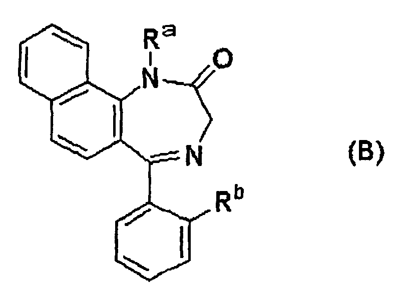 in which R^{a} is hydrogen or methyl and R^{b} is hydrogen or fluorine.

However, none of Non-patent publications 8 and 9 contain no teaching as to relationship between the disclosed compound and P2X₄ receptor antagonism, while both describe that the compound is employable as an agent for studying its binding action to a benzodiazepine receptor.

### [DISCLOSURE OF THE INVENTION]

The present invention has an object to provide 1,4-diazepin-2-one derivatives having the below-illustrated formulas (I) and (II) which show P2X₄ receptor antagonism.

The present invention resides in a compound having the following formula (I) or a pharmacologically acceptable salt thereof: 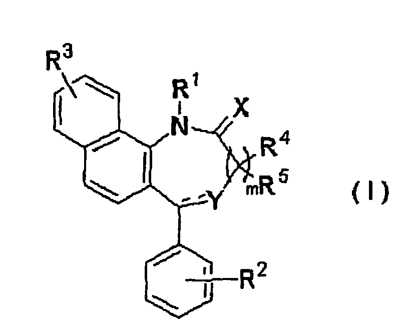 in which
X represents O, S or NH;
Y represents N or NR⁶ in which R⁶ is hydrogen or an alkyl group having 1-8 carbon atoms;
R¹ represents hydrogen, an alkyl group having 1-8 carbon atoms, an alkenyl group having 2-8 carbon atoms, an alkyl group having 1-8 carbon atoms which has 1-3 halogen substituents, or an alkyl group having a phenyl substituent;
R² represents an alkyl group having 1-8 carbon atoms, an alkoxy group having 1-8 carbon atoms, an alkyl group having 1-8 carbon atoms which has 1-3 halogen substituents, hydroxyl, nitro, amino, carboxyl, tetrazolyl, or cyano;
R³ represents hydrogen, an alkyl group having 1-8 carbon atoms, an alkoxy group having 1-8 carbon atoms, an alkyl group having 1-8 carbon atoms which has 1-3 halogen substituents, a halogen atom, hydroxyl, nitro, amino, carboxyl, tetrazolyl, or cyano;
each of R⁴ and R⁵ independently represents hydrogen, an alkyl group having 1-8 carbon atoms, or an alkyl group having 1-8 carbon atoms which has 1-3 halogen substituents;
m is 1 or. 2; and
the double line consisting of a solid line and a broken line represents a double bond in the case that Y is N and a single bond in the case that Y is NR⁶.

Further, the invention resides in a compound having the following formula (II) or a pharmacologically acceptable salt thereof: 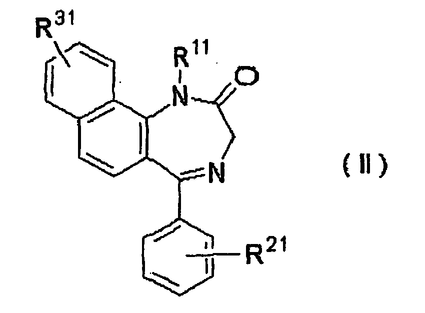 in which
R¹¹ represents hydrogen or an alkyl group having 1-8 carbon atoms;
R²¹ represents an alkyl group having 1-8 carbon atoms, an alkoxy group having 1-8 carbon atoms, an alkyl group having 1-8 carbon atoms which has 1-3 halogen substituents, or hydroxyl; and
R³¹ represents hydrogen or a halogen atom.

Furthermore, the invention resides in a P2X₄ receptor antagonist containing a compound of the formula (I) or (II) or its pharmacologically acceptable salt as an active ingredient.

Furthermore, the invention resides in an agent for preventing or treating neurogenic pain which contains a compound of the formula (I) or (II) or its pharmacologically acceptable salt as an active ingredient.

### [PREFERRED EMBODIMENTS OF THE INVENTION]

The invention is further described below.

In the formula (I) for the compound of the invention, the alkyl group having 1-8 carbon atoms for R¹, R², R³, R⁴, R⁵ and R⁶ can be methyl, ethyl, propyl, isopropyl, butyl, i-butyl, t-butyl, pentyl or hexyl.

The alkenyl group having 2-8 carbon atoms for R¹ can be vinyl or allyl.

The alkoxy group having 1-8 carbon atoms for R² and R³ can be methoxy, ethoxy, propoxy, isopropoxy, butoxy, i-butoxy, t-butoxy, pentyloxy or hexyloxy.

The halogen atom for R³ can be fluorine, chlorine or bromine.

The alkyl group having 1-8 carbon atoms which have 1-3 halogen substituents for R¹, R², R³, R⁴ and R⁵ can be methyl, ethyl, propyl, isopropyl, butyl or t-butyl which have 1-3 halogen substituents such as fluorine substituents, chlorine substituents or bromine substituents. Preferred are trifluoromethyl, chloromethyl, 2-chloroethyl, 2-bromoethyl and 2-fluoroethyl.

The alkyl group having a phenyl substituent for R¹ can be benzyl.

In the formula (I), same or different 1 to 3 R² and R³ can be attached to the benzene ring.

In the formula (II) for the compound of the invention, the alkyl group having 1-8 carbon atoms for R¹¹ and R²¹ can be methyl, ethyl, propyl, isopropyl, butyl, i-butyl, t-butyl, pentyl or hexyl.

The alkoxy group for R²¹ can be methoxy, ethoxy, propoxy, isopropoxy, butoxy, i-butoxy, t-butoxy, pentyloxy or hexyloxy.

The halogen atom for R³¹ can be fluorine, chlorine or bromine.

The alkyl group having 1-8 carbon atoms which have 1-3 halogen substituents for R²¹ can be methyl, ethyl, propyl, isopropyl, butyl or t-butyl which have 1-3 halogen substituents such as fluorine substituents, chlorine substituents or bromine substituents. Preferred are trifluoromethyl, chloromethyl, 2-chloroethyl, 2-bromoethyl and 2-fluoroethyl.

In the formula (II), same or different 1 to 3 R²¹ and R³¹ can be attached to the benzene ring.

As the compounds of the invention having the formula (I) or (II), the following compounds are preferred.
(1) The compound of the formula (I) or a pharmacologically acceptable salt thereof, in which m is 1.
(2) The compound of the formula (I) or the compound of (1) above, or a pharmacologically acceptable salt thereof, in which X is O.
(3) The compound of the formula (I) or the compound of (1) or (2) above, or a pharmacologically acceptable salt thereof, in which Y is N.
(4) The compound of the formula (I) or the compound of any one of (1) to (3) above, or a pharmacologically acceptable salt thereof, in which R¹ is hydrogen or an alkyl group having 1-8 carbon atoms.
(5) The compound of the formula (I) or the compound of any one of (1) to (3) above or a pharmacologically acceptable salt thereof, in which R¹ is hydrogen.
(6) The compound of the formula (I) or the compound of any one of (1) to (5) above, or a pharmacologically acceptable salt thereof, in which each of R⁴ and R⁵ is hydrogen.
(7) The compound of the formula (I) or the compound of any one of (1) to (6) above, or a pharmacologically acceptable salt thereof, in which R² is an alkyl group having 1-8 carbon atoms, an alkoxy group having 1-8 carbon atoms, an alkyl group having 1-8 carbon atoms which has 1-3 halogen substituents, or hydroxyl.
(8) The compound of the formula (I) or the compound of any one of (1) to (6) above, or a pharmacologically acceptable salt thereof, in which R² is an alkoxy group having 1-8 carbon atoms, or hydroxyl.
(9) The compound of the formula (I) or the compound of any one of (1) to (8) above, or a pharmacologically acceptable salt thereof, in which R³ is hydrogen or a halogen atom.
(10) The compound of the formula (I) or the compound of any one of (1) to (8) above, or a pharmacologically acceptable salt thereof , in which R³ is hydrogen.
(11) The compound of the formula (II) or a pharmacologically acceptable salt thereof, in which R¹¹ is hydrogen.
(12) The compound of the formula (II) or the compound of (11) above, or a pharmacologically acceptable salt thereof, in which R²¹ is an alkoxy group having 1-8 carbon atoms, or hydroxyl.
(13) The compound of the formula (II) or the compound of (11) or (12) above, or a pharmacologically acceptable salt thereof, in which R³¹ is hydrogen.

The pharmacologically acceptable salt of the compound of the formula (I) or (II) can be an alkali metal salt such as sodium salt, potassium salt or lithium salt.

The compound of the invention can be present in an optically active form or an optical isomer such as a racemic compound. These isomers can be included in the invention.

The schemes for synthesis of the compounds of the invention having the formula (I) or (II) are illustrated below.

### [Synthesis Process 1 (in the case of m=1 and Y=N)]

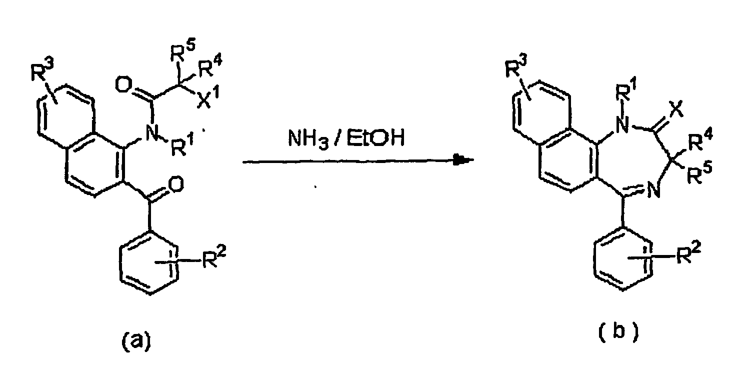

In the formulas, X¹ is a halogen atom such as bromine, and R¹, R², R³, R⁴ and R⁵ have the aforementioned meanings.

The compound of the invention having the formula (b) can be obtained by bringing the compound of the formula (a) into contact with a saturated ammonia-ethanol solution at room temperature.

The compound of the invention having the formula (b) also can be obtained by bringing the compound of the formula (a) into contact with an ammonia-dioxane solution in DMSO.

The starting compound, that is, the compound of the formula (a), can be obtained, for example, by the synthesis process illustrated below.

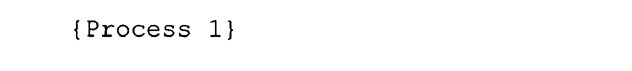

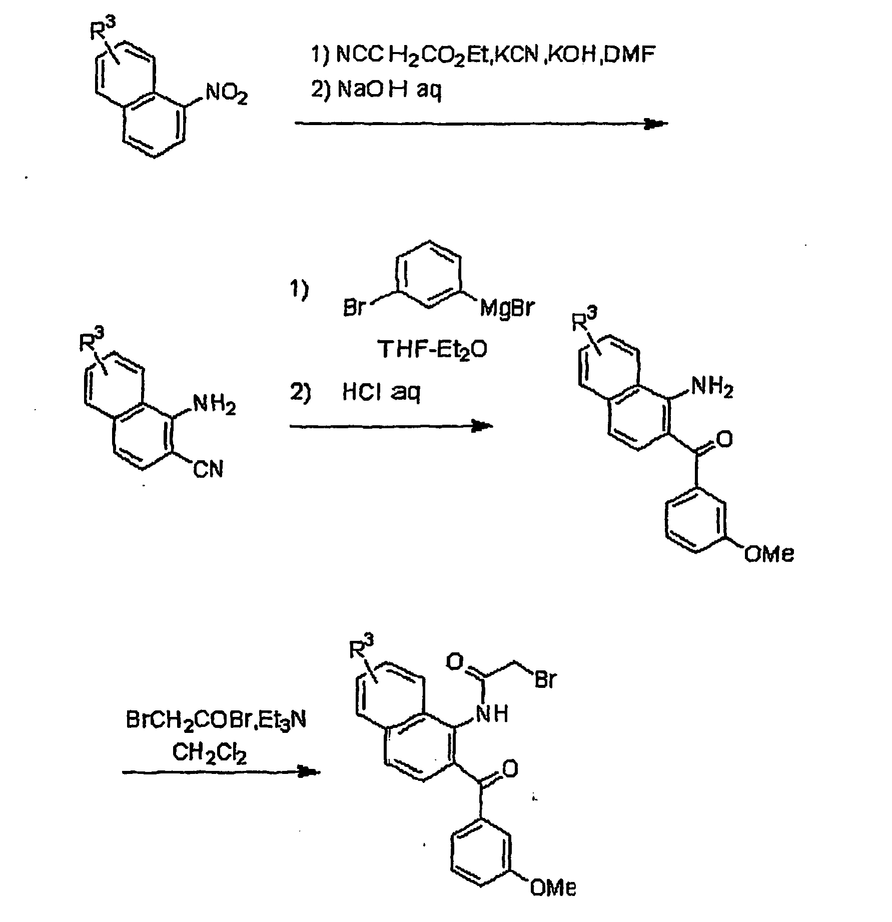

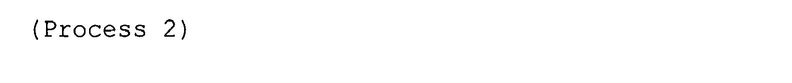

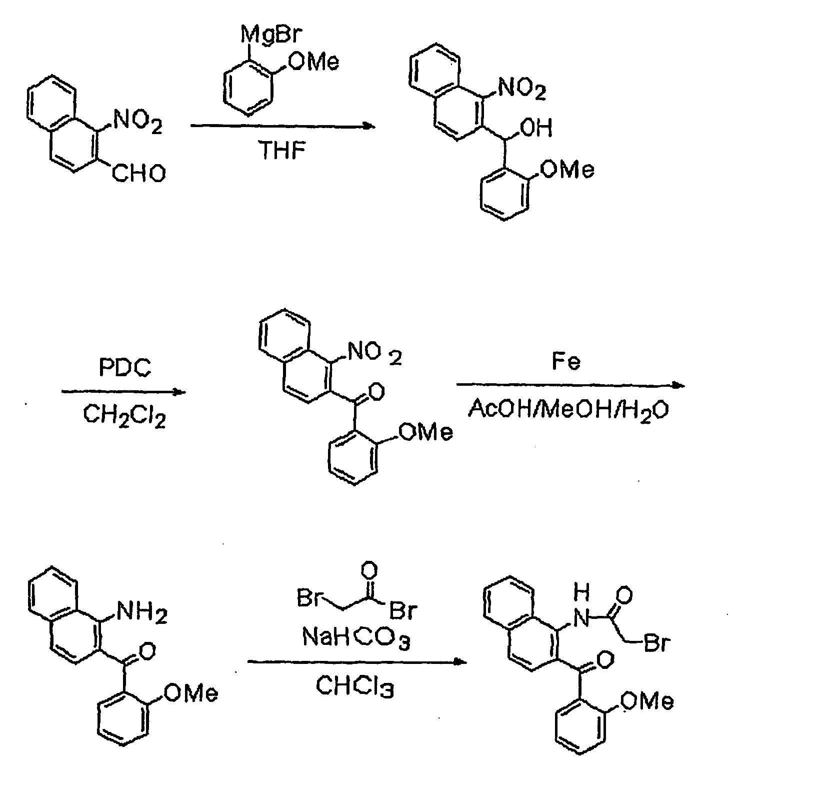

### [Synthesis Process 2 (in the case of R²=OH)]

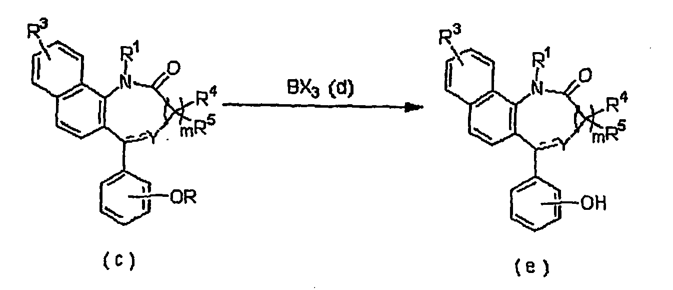

In the formulas, R is an alkyl group, X² is a halogen atom such as bromine, and R¹, R³, R⁴, R⁵, m, Y and double line consisting of a solid line and a broken line have the aforementioned meanings.

The compound of the invention having the formula (e) can be obtained by bringing the compound of the formula (d) into contact with the compound of the formula (c) in a solvent such as dichloromethane.

### [Synthesis Process 3 (in the case of R¹=alkyl)]

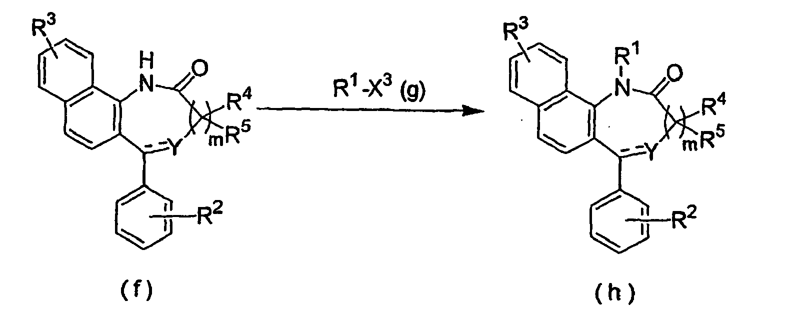

In the formulas, R¹ is an alkyl group, X³ is a halogen atom such as iodine, and R², R³, R⁴, R⁵, m, Y and double line consisting of a solid line and a broken line have the aforementioned meanings.

The compound of the invention having the formula (h) can be obtained by bringing the compound of the formula (g) into contact with the compound of the formula (f).

The compound of the invention having the formula (I) or (II) also can be obtained with reference to the working examples described hereinbelow and the descriptions of the aforementioned Patent publications and any other publications.

Examples of the compounds according to the invention are described in Tables 1 to 7.

### (1) Compounds of the following formula:

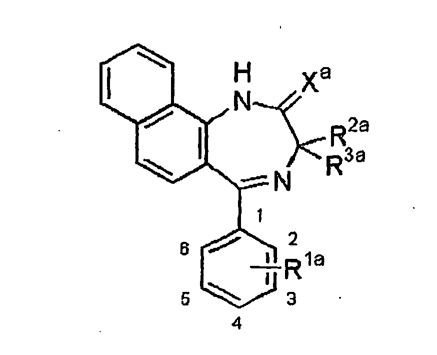

In the formula, each of R^{1a}, R^{2a}, R^{3a}, and X^{a} is that set forth in Tables 1 to 3.

**Table 1**

| R^{1a} | R^{2a}/R^{3a} | X^{a} |
|---|---|---|
| 3-OMe | H/H | O |
| 3-OH | H/H | O |
| 3-Me | H/H | O |
| 3-Et | H/H | O |
| 3-Ac | H/H | O |
| 3-CN | H/H | O |
| 3-CF₃ | Me/H | O |
| 3-OCF₃ | H/H | O |
| 3-CO₂Et | H/H | O |
| 3-CO₂H | H/H | O |
| 3-CO₂H | Et/H | O |
| 3-NO₂ | H/H | O |
| 3-CN | Me/H | O |

**Table 2**

| R^{1a} | R^{2a}/R^{3a} | X^{a} |
|---|---|---|
| 4-Me | Me/Me | S |
| 4-Et | Pr/H | NH |
| 4-Ac | H/H | S |
| 4-CF₃ | CF₃/H | O |
| 4-OMe | H/H | O |
| 4-OCF₃ | H/H | O |
| 4-CO₂Et | H/H | O |
| 4-CO₂H | H/H | O |
| 4-CO₂H | Et/H | O |
| 4-CN | H/H | O |
| 2-OH | H/H | O |
| 2-Me | Me/H | O |
| 3-CF₃ | Me/H | O |

**Table 3**

| R^{1a} | R^{2a}/R^{3a} | X^{a} |
|---|---|---|
| 2-OCF₃ | Me/H | S |
| 2-Ac | Pr/H | NH |
| 2-CO₂Et | H/H | O |
| 2-CO₂H | H/H | O |
| 2-CO₂H | Et/H | O |
| 2-CN | H/H | O |
| 2-OCF₃ | H/H | S |
| 3, 4-OH | H/H | O |
| 3, 5-Me | H/H | O |
| 2,5-Me | H/H | S |
| 3-Me, 4-OH | H/H | O |
| 2, 6-Me | Me/H | O |

### (2) Compounds of the following formula:

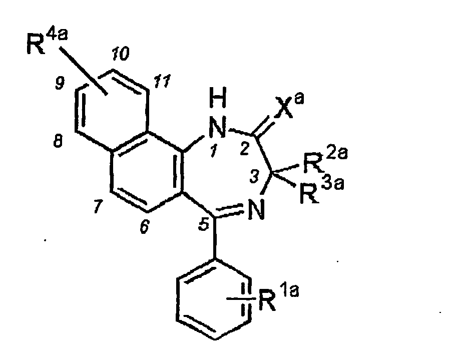

In the formula, each of R^{1a}, R^{2a}, R^{3a}, R^{4a} and X^{a} is that set forth in Tables 4 and 5.

**Table 4**

| R^{1a} | R^{2a}/R^{3a} | R^{4a} | X^{a} |
|---|---|---|---|
| 3-OH | H/H | 8-Br | O |
| 3-OH | H/H | 8,9-Cl | O |
| 3-OH | H/H | 10-F | O |
| 3-OH | H/H | 11-OH | O |
| 3-OH | H/H | 8CO₂H | O |
| 3-OH | Me/H | 9-Cl | O |
| 3-OH | Me/H | 10-CF₃ | S |
| 3-OH | Pr/H | 11-Me | NH |
| 3-OH | H/H | 8,9-OMe | S |
| 3-OH | H/H | 9-CN | O |
| 3-Me | H/H | 10-Br | O |
| 3-CN | H/H | 9,11-Cl | O |

**Table 5**

| R^{1a} | R^{2a}/R^{3a} | R^{4a} | X^{a} |
|---|---|---|---|
| 3-CO₂H | H/H | 8, 9-F | O |
| 3-OH, 4-Me | Me/H | 9-OH | O |
| 3,4-Me | Pr/H | 10-CO₂H | O |
| 2-CF₃ | Et/H | 11-Cl | S |
| 2,3-Me | Pr/H | 8-CF₃ | NH |
| 2,4-OMe | H/H | 9-Me | S |
| 3,4-OH | H/H | 9,10-OMe | O |
| 3,5-Me | H/H | 11-CN | O |
| 2,5-Me | H/H | 8-CF₃ | S |
| 3,5-OMe | H/H | 9-OH | O |
| 3,5-CO₂H | Me/H | 10-CO₂H | O |

### (3) Compounds of the following formula:

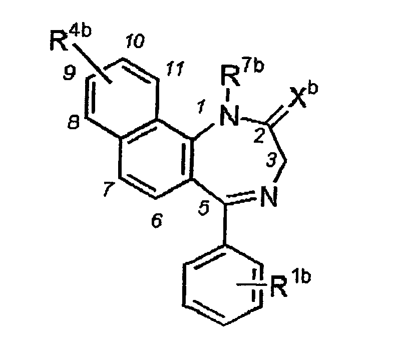

In the formula, each of R^{1b}, R^{4b}, R^{7b} and X^{b} is that set forth in Tables 6 and 7.

**Table 6**

| R^{1b} | R^{4b} | R^{7b} | X^{b} |
|---|---|---|---|
| 3-OH | H | Me | O |
| 3-Me | H | Et | O |
| 3-CO₂H | H | Me | O |
| 3-CF₃ | H | Pr | O |
| 3-CN | H | Bn | O |
| 3-Ac | H | Et | O |
| 3-OH | 9-Br | CH₂CH=CH₂ | S |
| 3-Me | 10-Cl | Me | NH |
| 3-CF₃ | 11-CO₂H | Et | S |
| 3-CO₂H | 11-OH | CH₂CH=CH₂ | O |
| 4-Me | 10-CO₂H | Pr | O |
| 4-OH | 10-CN | Me | O |
| 4-Ac | 10-CONH₂ | Et | O |
| 4-OCF₃ | 10-OH | Bn | O |

**Table 7**

| R^{1b} | R^{4b} | R^{7b} | X^{b} |
|---|---|---|---|
| 2-Me | 8-Br | CH₂CF₃ | O |
| 2-OMe | 8,9-Cl | Bn | O |
| 3,4-Me | 9-CF₃ | Me | O |
| 2,4-Me | 10-OH | Et | S |
| 2-CN | 11-CO₂H | CH₂CH=CH₂ | NH |
| 2-OCF3 | 10-Me | Pr | S |
| 3,4-OH | 10,11-Cl | Bn | O |
| 3,4-Me | 9-CF₃ | CH₂CF₃ | O |
| 2,6-Me | 8-OH | Me | S |
| 3, 5-OH | 8-CO₂H | Me | O |
| 3,5-OH | 9-CN | Et | O |

The pharmacological effects of the invention are described below.

The P2X₄ receptor antagonisms of the compounds according to the invention were determined in the following manner.

1321N1 cells were transfected human P2X₄ receptor-encoding expression vector using a FuGENE 6 transfection reagent (Roche). After cultivation of one week, it was confirmed that P2X₄ receptor was stably expressed. Cells were loaded with Fura2-AM calcium fluorescent dye (SIGMA) and the fluorescence changes were monitored using Aqua-Cosmos (Hamamatsu Photonics). ATP(10 µm)-induced maximal intramolecular calcium change was defined as 100% of control response to calculate the inhibition percentage of test compounds at each concentration. Test compounds were treated onto cells 10 minutes before ATP stimulation.

As is evident from the data of Examples 10 and 11, the compounds of the invention prepared in Examples 2, 4 and 9 show excellent P2X₄ receptor antagonism.

Therefore, it is considered that the compounds of the formulas (I) and (II) according to the invention which show P2X₄ receptor antagonism are effective as an agent for prevention and treatment of pains such as nociceptive pains, inflammatory pains and neurogenic pains. In other words, the compounds of the invention are effective to prevent or treat pains caused viral diseases such as herpes or osteoarthritis.

If desired, the agent for prevention or treatment according to the invention can be employed in combination with other medical agents such as opioide analgesics (morphine, fentanyl), sodium channel blockers (novocaine, lidocaine), NSAIDs (aspirin, ibuprofen).

The compound of the invention can be administered to human beings by ordinary administration methods such as oral administration or parenteral administration.

The compound can be granulated in ordinary manners for the preparation of pharmaceuticals. For instance, the compound can be processed to give pellets, granule, powder, capsule, suspension, injection, suppository, and the like.

For the preparation of these pharmaceuticals, ordinary additives such as vehicles, disintegrators, binders, lubricants, dyes, and diluents. As the vehicles, lactose, D-mannitol, crystalline cellulose and glucose can be mentioned. Further, there can be mentioned starch and carboxymethylcellulose calcium (CMC-Ca) as the disintegrators, magnesium stearate and talc as the lubricants, and hydroxypropylcellulose (HPC), gelatin and polyvinyl-pirrolidone (PVP) as the binders. The preparation of an injection can be made using solvents, stabilizers, dissolution-aids, suspensions, emulsifiers, soothing agents, buffers, preservatives, and the like.

The compound of the invention can be administered to an adult generally in an amount of approx. 0.01 mg to 100 mg a day by parenteral administration and 1 mg to 2,000 mg a day by oral administration. The dosage can be adjusted in consideration of age and conditions of the patient.

The invention is further described by the following non-limiting examples.

### [Example 1] 5-(3-Methoxyphenyl)-1,3-dihydro-2H-naptho-[1,2-e]-1,4-diazepin-2-one

### (1) 1-Amino-2-(3-methoxybenzoyl)naphthalene

A solution of 1-amino-2-naphthonitrile (1.00 g, 5.95 mmol) in anhydrous ether (20 mL) was dropwise added to a solution of 1M 3-methoxyphenylmagnesium bromide-tetrahydrofuran solution (17.8 mL, 17.8 mmol) for 10 minutes. The mixture was heated under reflux for one hour. The reaction mixture was poured into 2N hydrochloric acid (30 mL). After addition of methanol (5 mL), the mixture was stirred for 4 hours at room temperature. The mixture was neutralized by addition of potassium carbonate, and the neutralized ether was subjected to extraction with ether. The ether portion was washed with saturated brine and dried over anhydrous sodium sulfate. The dried mixture was placed under reduced pressure to distill the solvent off. The residue was purified by silica gel column chromatography (chloroform/hexane=4/1), to give the titled compound (1.54 g, yield 93%).
¹H NMR (CDCl₃, 400 MHz) δ: 3.85 (3H, s), 6.98 (1H, d, J=9Hz), 7.06 (1H, dd, J=2, 8Hz), 7.1-7.2 (2H, m), 7.37 (1H, t, J=8Hz), 7.4-7.6 (4H, m), 7.5-7.6 (1H, m), 7.74 (1H, d, J=8Hz), 7.97 (1H, d, J=8Hz).

### (2) 2-Bromo-N-[2-(3-methoxybenzoyl)naphthalen-1-yl]acetamide

Triethylamine (1.15 mL, 8.28 mmol) and bromoacetyl bromide (0.72 mL, 8.28 mmol) were added to a solution of the above-mentioned 1-amino-2-(3-methoxybenzoyl)naphthalene (1.53 g, 5.52 mmol) in anhydrous dichloromethane (25 mL) under cooling with ice, and the mixture was stirred for 2 hours at room temperature. Further, to the mixture were added triethylamine (0.77 mL) and bromoacetyl bromide (0.48 mL) under cooling with ice, and the mixture was stirred for one hour at room temperature. The reaction mixture was poured into saturated aqueous sodium hydrogen carbonate solution, and the aqueous mixture was subjected to extraction with chloroform. The organic portion was washed with saturated brine, dried over anhydrous sodium sulfate, and placed under reduced pressure to distill the solvent off. The residue was purified by silica gel column chromatography (chloroform/hexane=3/1 to 4/1), to give the titled compound (1.92 g, yield 88%).
¹H NMR (CDCl₃, 400 MHz) δ: 3.84 (3H, s), 3.97 (2H, s), 7.1-7.2 (1H, m), 7.3-7.5 (3H, m), 7.54 (1H, d, J=8Hz), 7.5-7.7 (2H, m), 7.84 (1H, d, J=8Hz), 7.8-8.0 (2H, m), 9.23 (1H, br s).

### (3) 5-(3-Methoxyphenyl)-1,3-dihydro-2H-naptho[1,2-e]-1,4-diazepin-2-one

A saturated ammonia-ethanol solution (20 mL) was added to the above-mentioned 2-bromo-N-[2-(3-methoxybenzoyl)naphthalen-1-yl]acetamide (1.92 g, 4.82 mmol), and the mixture was stirred overnight at room temperature. The reaction mixture was placed under reduced pressure to distill the solvent off. After addition of water, the residue was subjected to extraction with chloroform. The organic portion was washed with saturated brine, dried over anhydrous sodium sulfate, and placed under reduced pressure to distill the solvent off. The residue was purified by silica gel column chromatography (chloroform/methanol=98/2). The resulting crystalline product was suspended in ethyl acetate (4 mL). The suspension was heated under reflux for 30 hours and stirred for one hour at room temperature. The precipitated crystalline product was collected over a filter, to give the titled compound as a white crystalline product (605 mg, yield 40%).
m.p.: 218-220°C (decomp.)
¹H NMR (DMSO-d₆, 400 MHz) δ: 3.78 (3H, m), 3.7-3.9 (1H, m), 4.5-4.7 (1H, m), 7.02 (1H, d, J=8Hz), 7.08 (1H, dd, J=2, 8Hz), 7.15 (1H, br s), 7.28 (1H, d, J=8Hz), 7.35 (1H, t, J=8Hz), 7.6-7.8 (3H, m), 7.9-8.1 (1H, m), 8.3-8.4 (1H, m), 10.83 (1H, s).
IR (KBr, cm⁻¹): 3066, 1687, 1591, 1583, 1562, 1470, 1425, 1338, 1315, 1267, 1240, 1225, 1205, 1155, 1101, 1092, 1034, 1016, 995, 874, 825, 793, 758, 725, 701, 636, 571, 561, 490, 438.

### [Example 2] 5-(3-Hydroxyphenyl)-1,3-dihydro-2H-naptho-[1,2-e]-1,4-diazepin-2-one

To a solution of 5-(3-methoxyphenyl)-1,3-dihydro-2H-naptho[1,2-e]-1,4-diazepin-2-one (300 mg, 0.948 mmol) in anhydrous dichloromethane (9 mL) was added 1M boron tribromide-dichloromethane solution (1.9 mL, 1.90 mmol) under cooling with ice. The mixture was stirred overnight at room temperature. The reaction mixture was poured into saturated aqueous sodium hydrogen carbonate solution. After addition of chloroform, the mixture was stirred for 10 minutes at room temperature. An insoluble crystalline product was filtered off. The solution was subjected to extraction with chloroform. The organic portion was dried over anhydrous sodium sulfate and placed under reduced pressure to distill the solvent off. The residue was combined with the filtered crystalline product and purified by silica gel column chromatography (chloroform/methanol=96/4). The purified crystalline product was suspended in ethyl acetate (4 mL). The suspension was heated under reflux for 30 minutes and subsequently stirred at 0°C for one hour. The precipitated crystalline product was collected by filtration, to give the titled compound as a pale yellow crystalline product (151 mg, yield 53%).
m.p.: 267-269°C (decomp.)
¹H NMR (DMSO-d₆, 400 MHz) δ: 3.77 (1H, d, J=9Hz), 4.56 (1H, d, J=9Hz), 6.8-7.0 (2H, m), 6.98 (1H, br s), 7.23 (1H, t, J=8Hz), 7.28 (1H, d, J=8Hz), 7.6-7.8 (3H, m), 7.9-8.1 (1H, m), 8.2-8.4 (1H, m), 9.53 (1H, s), 10.81 (1H, s).
IR (KBr, cm⁻¹): 3183, 1680, 1595, 1574, 1514, 1485, 1404, 1362, 1311, 1279, 1217, 1151, 1041, 1020, 997, 894, 881, 818, 796, 754, 723, 706, 652, 563.

### [Example 3] 5-(4-Methoxyphenyl)-1,3-dihydro-2H-naptho-[1,2-e]-1,4-diazepin-2-one

The following intermediate compound and target compound were prepared by performing procedures similar to the procedures of Example 1.

### (1) 1-Amino-2-(4-methoxybenzoyl)naphthalene

¹H NMR (CDCl₃, 500 MHz) δ: 3.89 (3H, s), 6.9-7.0 (2H, m), 7.01 (1H, d, J=8Hz), 7.2-7.5 (2H, m), 7.5-7.6 (1H, m), 7.6-7.7 (2H, m), 7.75 (1H, d, J=8Hz), 7.96 (1H, d, J=8Hz).

### (2) 2-Cloro-N-[2-(4-methoxybenzoyl)naphthalen-1-yl]acetamide

¹H NMR (CDCl₃, 500 MHz) δ: 3.88 (3H, s), 4.14 (2H, s), 6.9-7.0 (2H, m), 7.51 (1H, d, J=8Hz), 7.6-7.7 (2H, m), 7.8-7.9 (3H, m), 7.9-8.0 (2H, m), 9.30 (1H, br s).

### (3) 5-(4-Methoxyphenyl)-1,3-dihydro-2H-naptho[1,2-e]-1,4-diazepin-2-one

¹H NMR (DMSO-d₆, 400 MHz) δ: 3.75 (1H, d, J=10Hz), 3.81 (3H, s), 4.53 (1H, d, J=10Hz), 6.99 (2H, d, J=9Hz), 7.29 (1H, d, J=9Hz), 7.50 (2H, d, J=9Hz), 7.6-7.8 (3H, m), 8.0-8.1 (1H, m), 8.3-8.4 (1H, m), 10.79 (1H, s).

### [Example 4] 5-(4-Hydroxyphenyl)-1,3-dihydro-2H-naptho-[1,2-e]-1,4-diazepin-2-one

The target compound was prepared by performing procedures similar to the procedures of Example 2.

¹H NMR (DMSO-d₆, 400 MHz) δ: 3.82 (1H, br s). 4.48 (1H, d, J=9Hz), 6.83 (2H, d, J=8Hz), 7.31 (1H, d, J=8Hz), 7.42 (1H, d, J=8Hz), 7.6-7.8 (3H, m), 8.0-8.1 (1H, m), 8.3-8.4 (1H, m), 10.89 (1H, br s).

### [Example 5] 5-(4-Methylphenyl)-1,3-dihydro-2H-naptho[1,2-e]-1,4-diazepin-2-one

The following intermediate compound and target compound were prepared by performing procedures similar to the procedures of Example 1.

### (1) 1-Amino-2-(4-methylbenzoyl)naphthalene

A mixture was obtained by performing procedures similar to the procedures of Example 1.

### (2) 2-Cloro-N-[2-(4-methylbenzoyl)naphthalen-1-yl]acetamide

¹H NMR (CDCl₃, 500 MHz) δ: 2.44 (3H, s), 4.13 (2H, s), 7.2-7.3 (2H, m), 7.53 (1H, d, J=8Hz), 7.6-7.7 (2H, m), 7.7-7.8 (2H, m), 7.85 (1H, d, J=8Hz), 7.9-8.0 (2H, m), 9.32 (1H, br s).

### (3) 5-(4-Methylphenyl)-1,3-dihydro-2H-naptho[1,2-e]-1,4-diazepin-2-one

¹H NMR (DMSO-d₆, 500 MHz) δ: 2.37 (3H, s), 3.78 (1H, d, J=10Hz), 4.56 (1H, d, J=10Hz), 7.2-7.3 (3H, m), 7.3-7.4 (2H, m), 7.4-7.8 (3H, m), 8.0-8.1 (1H, m), 8.3-8.4 (1H, m), 10.32 (1H, s).

### [Example 6] 5-(2-Methoxyphenyl)-1,3-dihydro-2H-naptho-[1,2-e]-1,4-diazepin-2-one

### (1) (2-Methoxyphenyl)(1-nitronaphthalen-2-yl)methanol

A solution of 1-nitronaphthalene-2-carboaldehyde (1.00 g, 5.00 mmol) in tetrahydrofuran (7 mL) was dropwise added to 1M 2-methoxyphenylmagnesium bromide-tetrahydrofuran solution (6.00 mL, 6.00 mmol) at a temperature of lower than 10°C. The mixture was stirred for 20 minutes at room temperature. The reaction mixture was poured into saturated aqueous ammonium chloride solution, and the aqueous mixture was subjected to extraction with ethyl acetate. The organic portion was successively washed with 1M hydrochloric acid and saturated aqueous sodium hydrogen carbonate solution, dried over anhydrous magnesium sulfate, and placed under reduced pressure to distill the solvent off. To the residue was added toluene, and the precipitated crystalline product was collected by filtration. The filtrate was purified by silica gel column chromatography (toluene/ethyl acetate=50/1), to give the titled compound (1.18 g in total, yield 76%).
¹H NMR (CDCl₃, 500 MHz) δ: 3.05 (1H, d, J=4Hz), 3.72 (3H, s), 6.35 (1H, d, J=4Hz), 6.84 (1H, d, J=8Hz), 7.00 (1H, t, J=8Hz), 7.30 (1H, td, J=2, 8Hz), 7.46 (1H, dd, J=2, 8Hz), 7.52 (1H, d, J=8Hz), 7.58 (1H, td, J=2, 8Hz), 7.63 (1H, td, J=2, 8Hz), 7.80 (1H, d, J=8Hz), 7.87 (1H, d, J=8Hz), 7.90 (1H, d, J=8Hz).

### (2) 2-(2-Methoxybenzoyl)-1-nitronaphthalene

Silica gel (6 g) and pyridinium dichromate (2.09 g, 5.54 mmol) were added to a solution of (2-methoxyphenyl)-(1-nitronaphthalen-2-yl)methanol (1.14 g, 3.69 mmol) in dichloromethane (20 mL). The mixture was stirred for 12 hours at room temperature. After addition of pyridinium dichromate (2.09 g, 5.54 mmol), the mixture was stirred at 30°C for 12 hours. After insolubles were filtered off, the filtrate was concentrated under reduced pressure. To the crystalline residue was added to toluene, and insolubles were collected by filtration, to give the titled compound (1.00 g, 88%).
¹H NMR (CDCl₃, 500 MHz) δ: 3. 58 (3H, s), 6.94 (1H, d, J=8Hz), 7.07 (1H, td, J=1, 8Hz), 7.5-7.6 (2H, m), 7.6-7.8 (3H, m), 7.9-8.0 (1H, m), 8.0-8.1 (2H, m).

### (3) 1-Amino-2-(2-methoxybenzoyl)naphthalene

Powdery iron (1.00 g) was added to a solution of 2-(2-methoxybenzoyl)-1-nitronaphthalene (966 mg, 3.14 mmol) in acetone (15 mL)/ethanol(15 mL)/water(3 mL). The mixture was stirred at 60°C for 10 hours. After addition of silica gel (10 g), the mixture was concentrated under reduced pressure to remove a volatile component. The residue was suspended in ethyl acetate (50 mL). To the suspension was added 1M aqueous sodium hydroxide solution, until the resulting mixture was turned basic. Insolubles were filtered over Celite. The organic portion was collected, washed with saturated aqueous sodium hydrogen carbonate, dried over anhydrous magnesium sulfate, and placed under reduced pressure to distill the solvent off, to give the titled compound (895 mg, quantitative).
¹H NMR (CDCl₃, 500 MHz) δ: 3.76 (3H, s), 6.90 (1H, d, J=8Hz), 7.00 (1H, d, J=8Hz), 7.04 (1H, td, J=2, 8Hz), 7.24 (1H, d, J=8Hz), 7.28 (1H, dd, J=2, 8Hz), 7.4-7.5 (2H, m), 7.56 (1H, td, J=2, 8Hz), 7.69 (1H, d, J=8Hz), 7.75 (2H, br s), 7.96 (1H, d, J=8Hz).

### (4) 2-Bromo-N-[2-(2-methoxybenzoyl)naphthalen-1-yl]acetamide

¹H NMR (CDCl₃, 500 MHz) δ: 3.68 (3H, s), 3.99 (2H, s), 6.9-7.0 (2H, m), 7.5-8.0 (8H, m), 9.85 (1H, br s).

### (5) 5-(2-Methoxyphenyl)-1,3-dihydro-2H-naphtho[1,2-e]-1,4-diazepin-2-one

¹H NMR (DMSO-d₆, 400 MHz) δ: 3.42 (3H, s), 7.0-7.1 (3H, m), 7.4-7.5 (2H, m), 7.6-7.7 (3H, m), 7.9-8.0 (1H, m), 8.3-8.4 (1H, m), 10.83 (1H, s).

### [Example 7] 5-(2-Hydroxyphenyl)-1,3-dihydro-2H-naptho-[1,2-e]-1,4-diazepin-2-one

The target compound was prepared by performing procedures similar to the procedures of Example 2.

¹H NMR (CDCl₃, 400 MHz) δ: 3.97 (1H, d, J=11Hz), 4.77 (1H, d, J=11Hz), 6.77 (1H, td, J=2.8Hz), 7.07 (1H, dd, J=2, 8Hz), 7.18 (1H, dd, J=2, 8Hz), 7.34 (1H, td, J=2, 8Hz), 7.53 (1H, d, J=8Hz), 7.7-7.8 (3H, m), 7.9-8.0 (1H, m)., 8.1-8.2 (1H, m), 8.49 (1H, br s), 13.74 (1H, s).

### [Example 8] 5-(3,4-Dimethoxyphenyl)-1,3-dihydro-2H-naptho[1,2-e]-1,4-diazepin-2-one

The following intermediate compound and target compound were prepared by performing procedures similar to the procedures of Example 6.

### (1) (3,4-dimethoxyphenyl) (1-nitronaphthalen-2-yl)-methanol

¹H NMR (CDCl₃, 500 MHz) δ: 2.58 (1H, d, J=3Hz), 3.86 (6H, s), 6.08 (1H, d, J=3Hz), 6.84 (1H, d, J=8Hz), 6.95 (1H, dd, J=2, 8Hz), 6.99 (1H, d, J=2Hz), 7.5-7.7 (3H, m), 7.75 (1H, d, J=8Hz), 7.89 (1H, d, J=8Hz), 7.94 (1H, d, J=8Hz).

### (2) 2-(3,4-Dimethoxybenzoyl)-1-nitronaphthalene

¹H NMR (CDCl₃, 400 MHz) δ: 3.95 (6H, s), 6.83 (1H, d, J=8Hz), 7.2-7.3 (1H, m), 7.5-7.6 (2H, m), 7.7-7.8 (2H, m), 8.01 (1H, d, J=8Hz), 8.13 (2H, t, J=8Hz).

### (3) 1-Amino-2-(3,4-dimethoxybenzoyl)naphthalene

¹H NMR (CDCl₃, 500 MHz) δ: 3.93 (3H, s), 3.96 (3H, s), 6.92 (1H, d, J=8Hz), 7.02 (1H, d, J=8Hz), 7.2-7.3 (2H, m), 7.4-7.6 (3H, m), 7.75 (1H, d, J=8Hz), 7.97 (1H, d, J=8Hz).

### (2) 2-Chloro-N-[2-(3,4-dimethoxybenzoyl)naphthalen-1-yl]acetamide

¹H NMR (CDCl₃, 500 MHz) δ: 3.93 (3H, s), 3.96 (3H, s), 4.15 (2H, s), 6.88 (1H, d, J=8Hz), 7.41 (1H, dd, J=2, 8Hz), 7.5-7.7 (4H, m), 7.86 (1H, d, J=8Hz), 7.9-8.0 (2H, m), 9.27 (1H, br s).

### (3) 5-(3,4-Dimethoxyphenyl)-1,3-dihydro-2H-naphtho[1,2-e]-1,4-diazepin-2-one

¹H NMR (DMSO-d₆, 400 MHz) δ: 3.75 (1H, d, J=10Hz), 3.77 (3H, s), 3.81 (3H,s), 4.54 (1H, d, J=10Hz), 6.92 (1H, d, J=8Hz), 6.98 (1H, d, J=8Hz), 7.3-7.4 4 (2H, m), 7.6-7.8 (3H, m), 8.0-8.1 (1H, m), 8.3-8.4 (1H, m), 10.78 (1H, s).

### [Example 9] 5-(3,4-Dihydroxyphenyl)-1,3-dihydro-2H-naptho[1,2-e]-1,4-diazepin-2-one

The target compound was prepared by performing procedures similar to the procedures of Example 2.
¹H NMR (DMSO-d₆, 400 MHz) δ: 3.72 (1H, d, J=10Hz), 4.47 (1H, d, J=10Hz), 6.74 (1H, d, J=8Hz), 6.78 (1H, d, J=8Hz), 7.05 (1H, s), 7.33 (1H, d, J=8Hz), 7.6-7.8 (3H, m), 7.9-8.1 (1H, m), 8.3-8.4 (1H, m), 9.12 (1H, br s), 9.36 (1H, s, br s), 10.75 (1H, br s).

### [Example 10] Pharmacological Experimental 1

### (Experimental procedures)

The P2X₄ receptor antagonisms of the compounds according to the invention were determined in the following manner.

1321N1 cells were transfected human P2X₄ receptor-encoding expression vector using a FuGENE 6 transfection reagent (Roche). After cultivation of one week, it was confirmed that P2X₄ receptor was stably expressed. Cells were loaded with Fura2-AM calcium fluorescent dye (SIGMA) and the fluorescence changes were monitored using Aqua-Cosmos (Hamamatsu Photonics). ATP(10 µm)-induced maximal intramolecular calcium change was defined as 100% of control response to calculate the inhibition percentage of test compounds at each concentration. Test compounds were treated onto cells 10 minutes before ATP stimulation.

### (Experimental result)

The compound of Example 2 showed an inhibition ratio of 69% at 10⁻⁵M. Accordingly, the compound of the invention of Example 2 has excellent P2X₄ receptor antagonism.

### [Example 11] Pharmacological Experimental 2

### (Experimental procedures)

The experimental procedures of Example 10 were repeated.

### (Experimental results)

The experimental results are set forth in Table 8.

**Table 8**

| Test compound | Inhibition ratio % (at 10⁻⁵M) |
|---|---|
| Example 4 | 53 |
| Example 9 | 63 |

As is evident from Table 8, the compounds of the invention of Examples 4 and 9 have excellent P2X₄ receptor antagonism.

## Claims

1. A compound having the following formula (I) or a pharmacologically acceptable salt thereof: 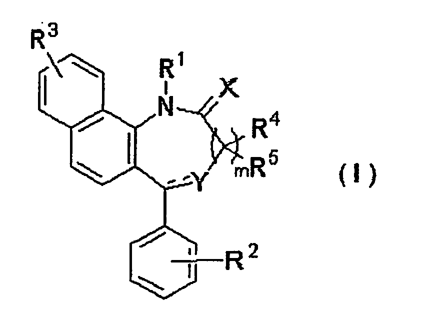 in which
X represents O, S or NH;
Y represents N or NR⁶ in which R⁶ is hydrogen or an alkyl group having 1-8 carbon atoms;
R¹ represents hydrogen, an alkyl group having 1-8 carbon atoms, an alkenyl group having 2-8 carbon atoms, an alkyl group having 1-8 carbon atoms which has 1-3 halogen substituents, or an alkyl group having a phenyl substituent;
R² represents an alkyl group having 1-8 carbon atoms, an alkoxy group having 1-8 carbon atoms, an alkyl group having 1-8 carbon atoms which has 1-3 halogen substituents, hydroxyl, nitro, amino, carboxyl, tetrazolyl, or cyano;
R³ represents hydrogen, an alkyl group having 1-8 carbon atoms, an alkoxy group having 1-8 carbon atoms, an alkyl group having 1-8 carbon atoms which has 1-3 halogen substituents, a halogen atom, hydroxyl, nitro, amino, carboxyl, tetrazolyl, or cyano;
each of R⁴ and R⁵ independently represents hydrogen, an alkyl group having 1-8 carbon atoms, or an alkyl group having 1-8 carbon atoms which has 1-3 halogen substituents;
m is 1 or 2; and
the double line consisting of a solid line and a broken line represents a double bond in the case that Y is N and a single bond in the case that Y is NR⁶.

2. The compound or a pharmacologically acceptable salt thereof according to claim 1, in which m is 1.

3. The compound or a pharmacologically acceptable salt thereof according to claim 1 or 2, in which X is O.

4. The compound or a pharmacologically acceptable salt thereof according to any one of claims 1 to 3, in which Y is N.

5. The compound or a pharmacologically acceptable salt thereof according to any one of claims 1 to 4, in which R¹ is hydrogen or an alkyl group having 1-8 carbon atoms.

6. The compound or a pharmacologically acceptable salt thereof according to any one of claims 1 to 4, in which R¹ is hydrogen.

7. The compound or a pharmacologically acceptable salt thereof according to any one of claims 1 to 6, in which each of R⁴ and R⁵ is hydrogen.

8. The compound or a pharmacologically acceptable salt thereof according to any one of claims 1 to 7, in which R² is an alkyl group having 1-8 carbon atoms, an alkoxy group having 1-8 carbon atoms, an alkyl group having 1-8 carbon atoms which has 1-3 halogen substituents, or hydroxyl.

9. The compound or a pharmacologically acceptable salt thereof according to any one of claims 1 to 7, in which R² is an alkoxy group having 1-8 carbon atoms, or hydroxyl.

10. The compound or a pharmacologically acceptable salt thereof according to any one of claims 1 to 9, in which R³ is hydrogen or a halogen atom.

11. The compound or a pharmacologically acceptable salt thereof according to any one of claims 1 to 9, in which R³ is hydrogen.

12. A compound having the following formula (II) or a pharmacologically acceptable salt thereof: 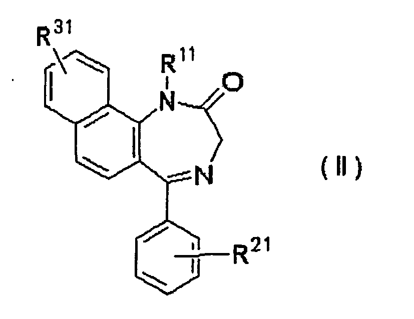 in which
R¹¹ represents hydrogen or an alkyl group having 1-8 carbon atoms;
R²¹ represents an alkyl group having 1-8 carbon atoms, an alkoxy group having 1-8 carbon atoms, an alkyl group having 1-8 carbon atoms which has 1-3 halogen substituents, or hydroxyl; and
R³¹ represents hydrogen or a halogen atom.

13. The compound or a pharmacologically acceptable salt thereof according to claim 12, in which R¹¹ is hydrogen.

14. The compound or a pharmacologically acceptable salt thereof according to claim 12 or 13, in which R²¹ is an alkoxy group having 1-8 carbon atoms, or hydroxyl.

15. The compound or a pharmacologically acceptable salt thereof according to any one of claims 12 to 14, in which R³¹ is hydrogen.

16. A compound selected from the following compounds or a pharmacologically acceptable salt thereof:
5-(3-methoxyphenyl)-1,3-dihydro-2H-naphtho[1,2-e]-1,4-diazepin-2-one;
5-(3-hydroxyphenyl)-1,3-dihydro-2H-naphtho[1,2-e]-1,4-diazepin-2-one;
5-(4-methoxyphenyl)-1,3-dihydro-2H-naphtho[1,2-e]-1,4-diazepin-2-one;
5-(4-hydroxyphenyl)-1,3-dihydro-2H-naphtho[1,2-e]-1,4-diazepin-2-one;
5-(4-methylphenyl)-1,3-dihydro-2H-naphtho[1,2-e]-1,4-diazepin-2-one;
5-(2-methoxyphenyl)-1,3-dihydro-2H-naphtho[1,2-e]-1,4-diazepin-2-one;
5-(2-hydroxyphenyl)-1,3-dihydro-2H-naphtho[1,2-e]-1,4-diazepin-2-one;
5-(3,4-dimethoxyphenyl)-1,3-dihydro-2H-naphtho[1,2-e]-1,4-diazepin-2-one; and,
5-(3,4-dihydroxyphenyl)-1,3-dihydro-2H-naphtho[1,2-e]-1,4-diazepin-2-one.

17. A P2X₄ receptor antagonist containing a compound or a pharmacologically acceptable salt thereof according to any one of claims 1 to 16 as an active ingredient.

18. An agent for preventing or treating neuropathic pain which contains a compound or a pharmacologically acceptable salt thereof according to any one of claims 1 to 16 as an active ingredient.
